# DEMANDE DE BREVET EUROPEEN

(11) **EP 1 360 949 A1**
(43) Date de publication de la demande: **12.11.2003**
(21) Numéro de dépôt: 03356074.9
(22) Date de dépôt: 30.04.2003
(51) Int. Cl.: A61F 2/36

(54) **Nouvelle prothèse totale de hanche**

(30) Priorité: 07.05.2002 FR 0205726
(71) Demandeur: Mosseri, Raphael, 94160 Saint Mande (FR); Merck Biomaterial France, 26903 Valence (FR)
(72) Inventeur: Mosseri, Raphael, 94160 Saint Mande (FR); de Witte, Gérard, 26300 Chateauneuf sur Isere (FR); Lakin, Ryan Cameron, Warsaw 46582 (US)
(74) Mandataire: Le Cacheux, Samuel L.R.

(57) **Abrégé**

La présente invention concerne le domaine de l'orthopédie.

La prothèse est caractérisée en ce que :
- la cupule comporte un méplat sommital **(13),** une portée tronconique divergente reliant le logement à la base de la cupule, et des moyens d'ancrage présentés par un épaississement (**15**) circonférentiel externe,
- la calotte comporte une surface extérieure sphérique (**12**) d'un diamètre inférieur à celui du logement de la cupule et des moyens d'ancrage présentés par un épaississement circonférentiel saillant intérieurement,
Application à une prothèse totale de hanche implantable par voie endoarticulaire.

## Description

La présente invention concerne le domaine de l'orthopédie et elle vise, plus particulièrement, celui des prothèses artificielles, du type total, qui sont destinées à suppléer aux défaillances des articulations naturelles du corps humain.

Plus spécifiquement encore, l'objet de l'invention concerne les prothèses totales de hanches, c'est-à-dire celles faisant intervenir les moyens techniques propres à remplacer les contreparties articulaires de l'articulation naturelle et dont l'une des deux au moins est lésée.

Il peut être considéré que, généralement, les prothèses totales de hanches comprennent deux éléments fondamentaux qu'il convient de pouvoir implanter.

Le premier élément peut être dénommé "implant cotyloïdien", en raison de sa forme en cupule généralement métallique qui est pourvue de moyens d'ancrage dans le cotyle naturel de l'os iliaque préalablement ou non réséqué. Une telle cupule présente, extérieurement, un logement globalement hémisphérique qui est, le plus fréquemment, garni d'un implant intermédiaire, généralement mais non exclusivement, en une matière plastique, telle que le polyéthylène.

Le second élément est dénommé "implant fémoral" et se trouve constitué par une tige dont la queue est destinée à être implantée dans le canal médulaire du fémur avec ou sans présence d'un ciment. Une telle queue est prolongée par un col prothétique surmonté d'une tête sphérique fémorale qui est destinée à se placer dans le logement de l'implant cotyloïdien.

Pour implanter une prothèse totale, il convient de procéder à une arthroplastie de la hanche, ayant pour but de pratiquer l'ablation du cartilage du cotyle naturel mais aussi du col fémoral, de manière à permettre l'implantation de la queue prothétique dans la diaphyse du fémur.

Une telle intervention implique, également, d'intervenir pour procéder à l'ablation quasiment totale de toute la capsule articulaire pour permettre une luxation convenable de l'articulation naturelle, en vue de procéder aux résections et ablations.

On conçoit que, pour intervenir dans de telles conditions, il est nécessaire d'exécuter préalablement une voie d'abord de dimensions suffisantes pour atteindre l'articulation.

Au total, une telle intervention, quoique bien maîtrisée actuellement, doit être considérée comme invasive, lourde, traumatisante et impliquant un processus de consolidation et de rééducation également lourd.

C'est dans le but de surmonter tout ou partie des inconvénients attachés à une telle pratique, que la technique antérieure a proposé des prothèses de hanche conçues pour éviter, lorsque cela est possible, la résection importante du cotyle et l'ablation de la tête du fémur, de manière à permettre une implantation par voie endo-articulaire laissant subsister l'intégrité de la capsule articulaire environnementale et favorisant, en conséquence, le maintien d'une bonne vascularisation générale.

Une telle prothèse qui peut être illustrée par l'enseignement du brevet FR 96 07452 se caractérise par l'existence de deux éléments constitutifs qui ont pour caractéristique de pouvoir être introduits dans l'espace articulaire par une voie d'accès antéro-externe beaucoup moins invasive en tant que telle et dans ses conséquences que la voie d'abord traditionnelle pour la mise en place ou l'implantation d'une prothèse totale du type rappelé précédemment.

Ces deux éléments constitutifs comprennent une cupule destinée à être implantée, après préparation partielle, dans la cavité acétabulaire par impaction, et une calotte complémentaire destinée à être adaptée après résection partielle sur la tête du fémur naturel, en étant immobilisée aussi par impaction au moyen d'un matériel i ancillaire approprié.

Toutefois, cette technique décrite dans le brevet FR 96-07452, implique de disposer d'un matériel ancillaire spécifique qui ne peut pas être considéré comme totalement satisfaisant, en raison de la conformation des deux éléments constitutifs de la prothèse.

Un but de l'invention est de proposer des modifications constructives des éléments constitutifs de la prothèse, afin de favoriser la mise en place par une technique moins invasive , un meilleur ancrage et une meilleure coopération entre des éléments constitutifs, de façon à restaurer, d'une manière optimale, l'articulation naturelle lésée.

Un autre but de l'invention est de proposer des implants de volume réduit.

C'est justement l'objet de l'invention que de proposer une nouvelle prothèse totale de hanche du type endo-articulaire afin de satisfaire aux besoins d'amélioration précités.

Pour atteindre les objectifs ci-dessus, la prothèse totale de hanche conforme à l'invention et qui est du type comprenant, d'une part, un implant cotyloïdien de forme générale en cupule délimitant intérieurement un logement sensiblement semi-sphérique et comportant extérieurement des moyens d'ancrage dans la cavité acétabulaire de l'os iliaque et, d'autre part, un implant fémoral en forme générale de calotte définissant extérieurement une surface sphérique apte à coopérer avec le logement et intérieurement une cavité sensiblement semi-sphérique comportant des moyens d'ancrage sur la tête fémorale, est caractérisée en ce que :
- la cupule comporte :
   - un méplat sommital sur sa face extérieure,
   - une portée tronconique divergente reliant le logement au plan diamétral définissant 1a base de la cupule,
   - et des moyens d'ancrage présentés par un épaississement circonférentiel externe établi à partir dudit plan diamétral,
- la calotte comporte :
   - une surface extérieure sphérique d'un diamètre inférieur à celui du logement de la cupule d'une mesure comprise entre 0,05 et 0,12 mm,
   - et des moyens d'ancrage présentés par un épaississement circonférentiel saillant intérieurement et reliant ladite surface au plan diamétral définissant la base de la calotte,

Diverses autres caractéristiques de l'invention ressortent de la description faite ci-dessous en référence aux dessins annexés qui montrent, à titre d'exemples non limitatifs, des formes de réalisations de l'objet de l'invention.
La **fig. 1** est une vue schématique illustrant une articulation coxo-fémorale sur laquelle une implantation d'une prothèse totale conforme à l'invention doit intervenir.
La **fig. 2** est une élévation éclatée, en partie arrachée, illustrant les différents éléments constitutifs de la prothèse selon l'invention.
La **fig. 3** est une coupe correspondant à l'un des éléments constitutifs selon la **fig. 2.**
La **fig. 4** est une élévation du même élément constitutif mais illustrant une variante de réalisation.
La **fig. 5** est une vue partielle montrant, à plus grande échelle, un détail constructif de l'élément selon la **fig. 4.**
La **fig. 6** est une perspective correspondant à la **fig. 2** et faisant apparaître, plus clairement, certains détails constructifs.

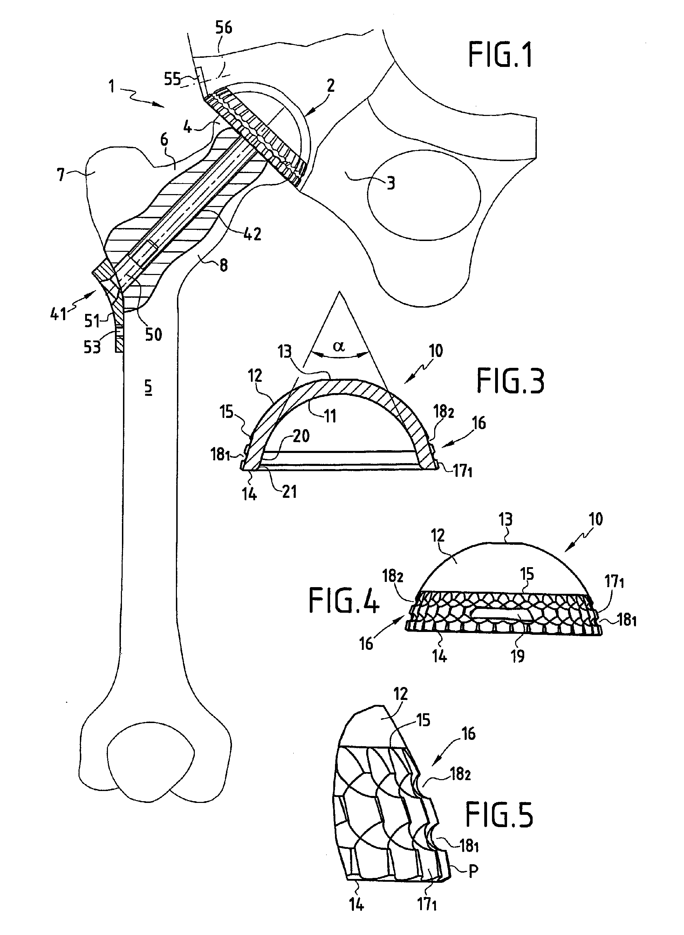

La **fig. 1** montre, de façon schématique, une articulation coxo-fémorale **1** composée, de manière simplifiée, de la cavité acétabulaire **2** présentée par l'os iliaque **3** et de la tête fémorale **4** d'un fémur **5** dont la partie proximale forme naturellement le col fémoral **6** s'étendant sensiblement à partir des deux protubérances naturelles **7** et **8** dénommées grand et petit trochanters.

L'objet de l'invention est de suppléer à la défaillance ou à la lésion des parties articulaires en coopération et qui sont la tête du fémur **5** et le cotyle tapissant ou définissant la cavité acétabulaire **2**.

La prothèse totale de hanche conforme à l'invention est destinée à être implantée dans tous les cas où l'articulation naturelle peut être considérée comme lésée au droit des surfaces articulaires, sans que d'autres désordres importants impliquent des travaux de restauration ou de restructuration pour ce qui concerne le col 6, la capsule articulaire, etc.

En d'autres termes, la prothèse totale de hanche conforme à l'invention doit être considérée comme implantable après rectification ou, plus précisément, résection de faible importance des cartilages de la cavité acétabulaire et de la tête du fémur, par des moyens connus dans la technique et qui ne font pas partie directement de l'invention.

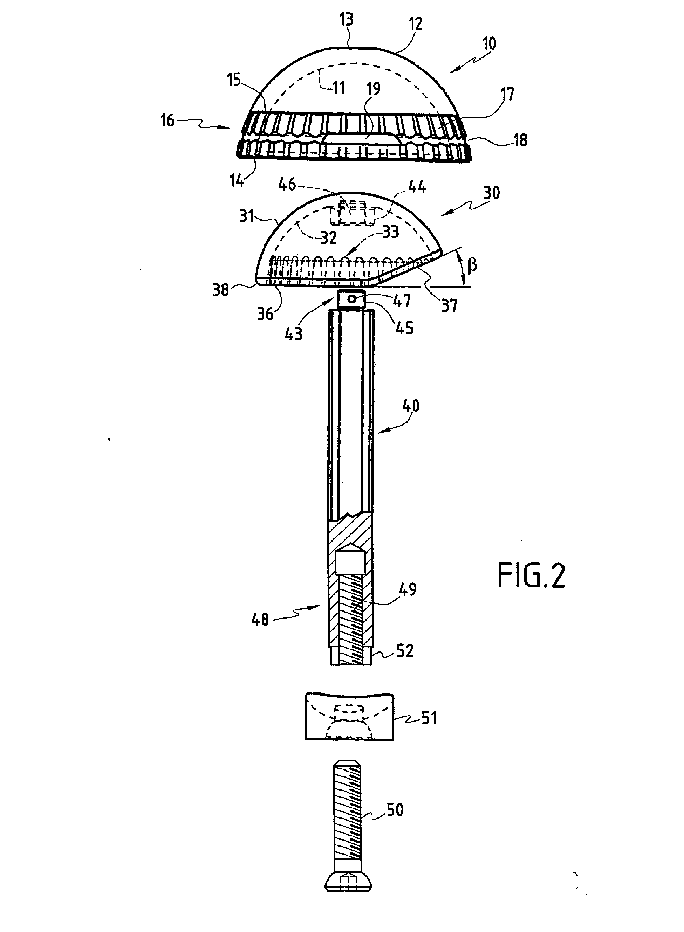

La prothèse totale de hanche conforme à l'invention comprend une première partie, dénommée implant cotyloïdien, désignée dans son ensemble par la référence 10 à la fig. 2. Cet implant cotyloïdien présente une forme générale en cupule délimitant intérieurement un logement sensiblement semi-sphérique 11 et comportant une surface extérieure 12 de forme générale sensiblement semi-sphérique.

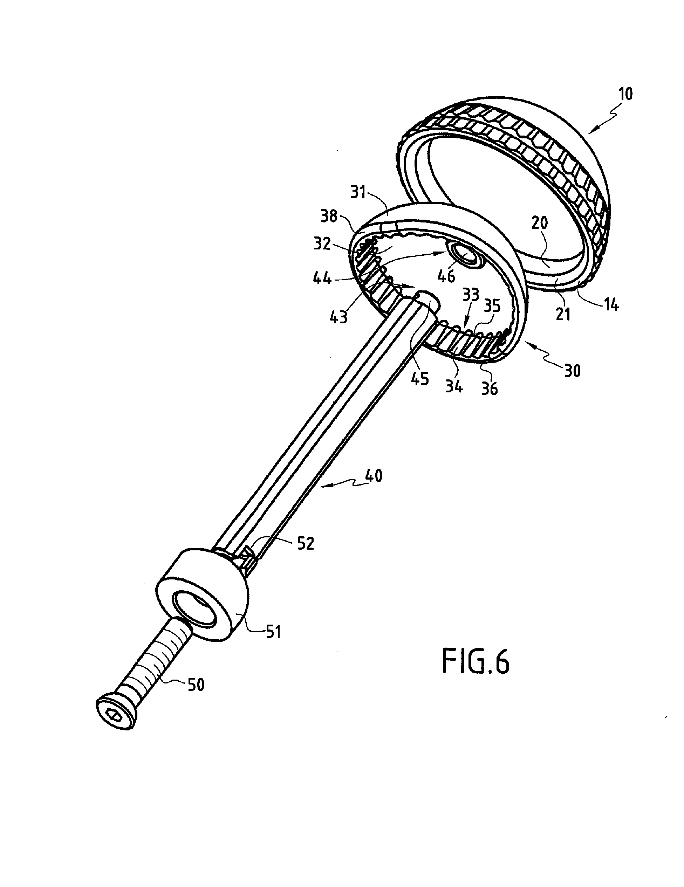

Au sens de l'invention, la cupule **10** présente avantageusement la caractéristique constructive de posséder un méplat sommital **13** sur sa surface extérieure **12** (**fig. 3**) et d'être délimitée, à sa base opposée audit méplat, par un plan diamétral **14.**

Selon une autre caractéristique constructive, la surface sensiblement semi-sphérique **12** est raccordée à la base **14** par un épaississement **15** dans lequel sont aménagés, formés ou autrement exécutés des moyens d'ancrage **16** destinés à être amenés à pénétrer dans l'os ou la partie osseuse ou spongieuse de la cavité acétabulaire réséquée, par impaction ou analogue. Ces moyens d'ancrage **16** permettent d'assurer la fixation primaire de la cupule et une meilleure réhabilitation osseuse en raison de l'accroissement de la surface de contact qu'ils offrent. Ces moyens d'ancrage **16** sont constitués, dans l'exemple selon la **fig. 2,** par des crans **17** ménagés selon des plans méridiens, de façon à délimiter des alvéoles favorables à une reconstitution osseuse. Les crans **17** peuvent aussi être complétés par la présence d'une rainure **18** pratiquée selon un plan parallèle, de telle manière que les moyens d'ancrage présentent une conformation réservant des alvéoles aptes à accueillir le développement osseux de reconstitution après impaction. Les **fig. 3, 4** et **5** montrent une variante de réalisation préférée dans laquelle l'épaississement présente une épaisseur décroissant depuis le plan diamétral **14,** par exemple deux rainures **18**_{**1**} et **18**_{**2**} et des crans **17**_{**1**} délimitant ensemble des plots **P** de largeur et de hauteur variant dans le même sens depuis le plan **14,** pour favoriser des conditions d'impaction meilleures et délimiter des sites d'accueil encore plus favorables à la reconstitution osseuse.

Le méplat sommital **13** a pour fonction principale d'éviter en premier lieu lors de l'impaction un contact avec le fond de la cavité acétabulaire et de privilégier ainsi la pénétration préférentielle des moyens d'ancrage **16**. Ce méplat **13** permet aussi de favoriser l'engagement de la cupule dans la cavité.

L'épaississement **15** est mis à profit pour faire apparaître une ou, de préférence, plusieurs conformations locales **19** qui ont pour vocation de permettre l'adaptation d'un matériel ancillaire de préhension facilitant le maintien, l'engagement, la disposition et la mise en place dans l'espace inter-articulaire au cours de l'intervention. Les conformations **19** sont, de préférence, constituées par des empreintes à caractère négatif et sont, préférentiellement encore, au nombre de deux en étant diamétralement opposées.

L'implant cotyloïdien **10** se caractérise également par la forme sensiblement semi-sphérique du logement **11** qui se raccorde au plan diamétral **14** par une portée tronconique divergente **20** qui est caractérisée par un angle au centre **α** pouvant être compris entre 25 et 35°. Un tel angle **α** possède, de préférence, une valeur égale à 30°. La portée tronconique, qui se raccorde de manière tangentielle au logement **11**, est également raccordée au plan diamétral **14** par un congé **21**.

Le second élément constitutif de la prothèse totale selon l'invention est constitué par un implant fémoral **30** en forme de calotte sensiblement semi-sphérique qui définit une surface extérieure **31** apte à coopérer avec le logement **11**. A cet effet, la surface extérieure **31** est définie par un diamètre qui est inférieur à celui définissant le logement **11** d'une mesure pouvant être comprise entre 0,05 et 12/10^{ème} de millimètre. Cette différence de diamètre, alliée à la présence de la portée tronconique 20 permet d'établir un accouplement articulaire faisant intervenir un simple contact de type ponctuel entre les surfaces **11** et **12** en contact et supprimant tout risque de coincement et de grippage sous charge.

La calotte **30** délimite, intérieurement, une cavité **32** destinée à emboîter la tête fémorale réséquée sur laquelle la calotte **30** est adaptée par des moyens d'ancrage **33** apparaissant, plus précisément, à la **fig. 6.** Ces moyens d'ancrage **33** sont constitués par des crans **34** établis selon des plans méridiens dans un épaississement circonférentiel **35** qui raccorde la cavité **32** à un plan diamétral **36** définissant la base de la calotte **30**. Les crans **34** peuvent être purement méridiens ou être complétés, comme dans le cas de l'implant cotyloïdien, par une ou plusieurs rainures du type de la ou des rainures **18.** Il doit donc être compris que l'épaississement **35** représente une saillie circonférentielle interne, de telle sorte que les crans pratiqués laissent également subsister entre eux des arêtes favorisant l'immobilisation de la calotte sur la tête fémorale, de préférence par impaction. Comme pour la cupule, l'épaississement **35** peut présenter une épaisseur décroissant depuis le plan diamétral **36**, de façon que les crans **34** définissent des sites d'accueil de profondeur variant progressivement.

Dans une forme de réalisation préférée, le plan diamétral 36 couvre sensiblement la demi-circonférence de la base et se trouve complété par une échancrure **37** qui peut être considérée comme définie par un pan incliné faisant, avec le plan **36**, un angle (β compris entre 15 et 25° . Quelle que soit la forme de la calotte, la base est avantageusement raccordée à la surface extérieure **31** par un congé **38**.

L'échancrure **37** permet de réduire le volume de la calotte **30** et de favoriser, d'une part, l'engagement dans l'espace interarticulaire et, d'autre part, le maintien de la capsule articulaire.

Les deux éléments constitutifs **10** et **30** de la prothèse peuvent être réalisés en un même matériau de base qui peut être choisi parmi notamment le chrome-cobalt, la céramique à base d'alumine, la céramique à base de zircone ou encore en couple de matériaux complémentaires.

La prothèse selon l'invention peut aussi comporter un troisième élément constitué par une tige **40** de fixation intra-cervicale destinée à être mise sous contrainte de traction entre la calotte **30** et une butée **41** de serrage et d'appui contre la corticale externe du fémur.

Une telle tige **40** est destinée à être logée dans un tunnel intra-cervical **42** ménagé dans la partie épiphysaire proximale du fémur **5** plus particulièrement dans l'axe du col **6,** de façon à s'ouvrir au sommet de la tête hémisphérique fémorale réséquée et à la base du grand trochanter **7.** La tige de fixation intra-cervicale comprend, à une extrémité proximale, des moyens **43** de liaison temporaire, de préférence freinée, avec des moyens complémentaires **44** présentés par la cavité **32**. Par moyens de liaison temporaire, il convient de considérer tout moyen technique susceptible de répondre à la fonctionnalité recherchée et, par exemple, il peut s'agir, pour la tige **40**, d'un embout fileté **45** et, pour la cavité **32**, d'un puits présentant un taraudage borgne **46**. Dans un tel exemple, il est avantageux de faire comporter, à l'embout **45**, un trou traversant diamétral **47** permettant la mise en place d'un segment de jonc en matière plastique appropriée, dont la longueur est excédentaire par rapport au diamètre de l'embout **45**, de manière à constituer un frein, notamment au dévissage libre de la tige **40.**

Tout moyen technique équivalent fonctionnellement peut être envisagé en remplacement de l'embout **43** et du puits **46.**

La tige **40**, qui peut être doublement méplate, est pourvue d'une partie terminale distale **48** présentant axialement un taraudage borgne **49** destiné à la mise en place d'une vis **50**, dite de mise en contrainte de traction de la tige **40** et traversant une cale d'adaptation **51** présentant, comme cela ressort de la **fig. 1,** une forme conjuguée à la forme locale de la corticale fémorale.

La partie terminale distale **48** est, avantageusement, pourvue aussi d'une fente **52**, du type en tournevis, permettant d'assurer le montage de la tige **40** sur la calotte **30** dans le cas où les moyens **43** sont du type à filetage, ainsi que le démontage éventuel.

Il doit être noté que le trou borgne taraudé **49** peut également être mis à profit pour l'adaptation de moyens d'impaction par contrainte de traction, du type à percussion.

La **fig. 1** montre que la cale **51** peut posséder une forme allongée et présenter un trou **53** pour le montage d'une vis de fixation primaire.

La **fig. 1** montre également que la cupule peut aussi comporter, de façon monobloc, à partir de la base, une patte **55** permettant après impaction une fixation dans l'os iliaque par une vis **56.**

Plusieurs méthodes d'implantation de la prothèse, selon l'invention, peuvent être indifféremment adaptées. Toutefois, afin de bénéficier au maximum du caractère mini-invasif qu'offre une telle prothèse, une méthode d'implantation préférée consiste à agir comme décrit ci-après.

La pose est dite par voie endo-articulaire c'est-à-dire que l'articulation n'est pas luxée, toutes les étapes étant effectuées avec l'articulation en l'état et en particulier le fraisage cotyloidien et fémoral.

L'installation du patient est en décubitus dorsal, inclinaison à 45°, pour effectuer la voie d'abord externe par incision d'environ 4 à 5 centimètres en sus trochantérien et en avant (type HARDINGS). La capsule articulaire est abordée par incision en respectant son intégrité et la plupart des vaisseaux qui irriguent le col fémoral. La synoviale sera conservée dans la mesure où elle ne présente pas de signes pathologiques.

L'articulation coxo-fémorale est alors sous contrôle de la vue et un nettoyage des ostéophytes peut être pratiqué. Le ligament rond est sectionné et une distraction primaire de la tête fémorale par rapport à la cavité acétabulaire est pratiquée à l'aide d'un écarteur approprié. L'écart obtenu est d'environ 3 cm, le fraisage des surfaces articulaires sera réalisé sans luxer l'articulation.

Le tunnel transfémoral **12** est réalisé pour déboucher au centre de la tête fémorale.

Une fraise hémisphérique est acheminée par l'incision initiale et une tige d'entraînement est introduite par le tunnel transcervical, les deux pièces sont accouplées et entraînées par un système soit manuel soit moteur.

Le fraisage de la cavité est réalisé en imprimant une poussée sur l'ensemble fraise - porte fraise, la cavité est fraisée jusqu'à ce que le cartilage ait disparu et que l'os sous-chondral saigne.

Les instruments sont alors retirés en pratiquant les mêmes gestes à l'envers.

Une fraise hémisphérique en forme de cloche est alors introduite et le porte fraise est acheminé par le tunnel transcervical. Après correction, le fraisage de la tête est réalisé en exerçant une traction sur l'ensemble fraise - porte fraise. Le fraisage est réalisé en os sous-chondral et les instruments sont retirés par la même voie.

L'espace entre la tête et la cavité acétabulaire est maintenant d'environ 5 centimètres, ce qui est suffisant pour l'introduction des implants prothétiques.

Le cotyle est acheminé par la voie supérieure et sa fixation peut être assurée avec ou sans ciment.

Lorsque l'implant est positionné, un impacteur passant par le tunnel est actionné et le cotyle est impacté dans la cavité osseuse. Le matériel est retiré en procédant à l'inverse de la phase de mise en place.

La calotte fémorale est alors positionnée sur la tête osseuse fraisée, la tige prothétique transcervicale est connectée à la calotte prothétique grâce au système de pas de vis. Un ensemble compacteur avec masse coulissante est connecté à la tige et des efforts de traction sont appliqués visant à impacter la calotte sur le moignon osseux.

Après enlèvement de l'ensemble compacteur, la cale **51** est positionnée sur la corticale externe du fémur et elle est solidarisée à la tige grâce à la vis de traction **50** qui assure la compression de la calotte sur le moignon de la tête fémorale.

L'arthroplastie a été réalisée sans luxer l'articulation coxo fémorale, respectant ainsi l'environnement capsulaire, vasculaire, musculaire et dans certains cas la membrane synoviale est conservée, ce qui contribue à une production de liquide articulaire servant à lubrifier les deux pièces en friction.

L'articulation est fermée en fermant la capsule.

L'intérêt d'une telle technique est de :
- respecter l'environnement de l'articulation et assurer une meilleure fonction (stabilité, usure)
- limiter les saignements per-opératoires,
- permettre une hospitalisation plus courte,
- lutter contre l'infection,
- réaliser des économies en terme de coût de santé.

L'invention n'est pas limitée aux exemples décrits et représentés car diverses modifications peuvent y être apportées sans sortir de son cadre.

## Revendications

1. Prothèse totale de hanche du type comprenant :
• d'une part, un implant cotyloïdien **(10)** de forme générale en cupule délimitant intérieurement un logement **(11)** sensiblement semi-sphérique et comportant extérieurement des moyens d'ancrage **(16)** dans la cavité acétabulaire de l'os iliaque,
• et, d'autre part, un implant fémoral **(30)** en forme générale de calotte définissant extérieurement une surface sphérique **(31)** apte à coopérer avec le logement et intérieurement une cavité **(32)** sensiblement semi-sphérique comportant des moyens **(33)** d'ancrage sur la tête fémorale,
**caractérisée en ce que** :
• la cupule comporte
- un méplat sommital **(13)** sur sa surface extérieure,
- une portée tronconique divergente **(20)** reliant le logement au plan diamétral **(14)** définissant la base de la cupule,
- et des moyens d'ancrage présentés par un épaississement **(15)** circonférentiel externe établi à partir dudit plan diamétral,
• la calotte comporte
- une surface extérieure sphérique **(12)** d'un diamètre inférieur à celui du logement de la cupule d'une mesure comprise entre 0,05 et 0,12 mm,
- et des moyens d'ancrage présentés par un épaississement **(35)** circonférentiel saillant intérieurement et reliant ladite surface au plan diamétral **(36)** définissant la base de la calotte,

2. Prothèse selon la revendication 1, **caractérisée en ce que** la portée tronconique **(20)** de la cupule est définie par un angle au centre compris entre 25 et 35°.

3. Prothèse selon la revendication 1 ou 2, **caractérisée en ce que** la portée tronconique se raccorde tangentiellement au logement.

4. Prothèse selon l'une des revendications 1 à 3 ? **caractérisée en ce que** la portée tronconique est raccordée au plan diamétral par un congé **(21)**.

5. Prothèse selon la revendication 1, **caractérisée en ce que** les moyens d'ancrage sont formés par des crans **(17)** ménagés dans l'épaississement selon des plans méridiens.

6. Prothèse selon la revendication 5, **caractérisée en ce que** les moyens d'ancrage comprennent, en outre, au moins une rainure parallèle **(18)**.

7. Prothèse selon la revendication 1, 5 ou 6, **caractérisée en ce que** les moyens d'ancrage comprennent une patte **(55)** percée s'étendant solidairement à partir du plan diamétral et réservée à la mise en place d'au moins une vis **(56)** de fixation osseuse.

8. Prothèse selon la revendication 1, **caractérisée en ce que** l'épaississement **(15)** présente des empreintes **(19)** pour l'adaptation de moyens de préhension.

9. Prothèse selon la revendication 1 ou 8, **caractérisée en ce que** la surface extérieure de la calotte est raccordée au plan diamétral par un congé **(38).**

10. Prothèse selon la revendication 1, **caractérisée en ce que** les moyens d'ancrage de la calotte sont formés par des crans **(34)** ménagés dans l'épaississement **(35)** selon des plans méridiens.

11. Prothèse selon la revendication 1, **caractérisée en ce que** le plan diamétral **(36)** de la calotte couvre sensiblement la demi-circonférence et se trouve raccordé à une échancrure **(37)** définie par un pan incliné.

12. Prothèse selon la revendication 1, **caractérisée en ce qu'**elle comprend, en outre, une tige **(40)** de fixation intracervicale destinée à être mise sous contrainte de traction entre la calotte **(30)** et une cale **(51)** de serrage et d'appui contre le grand trochanter du fémur.

13. Prothèse selon la revendication 12, **caractérisée en ce que** la tige est liée à la calotte par des moyens d'adaptation amovible du type à freinage **(43-44).**

14. Prothèse selon la revendication 12 ou 13, **caractérisée en ce que** la tige **(40)** comporte, en partie distale opposée à la calotte, des moyens de serrage et d'appui amovibles comprenant une cale **(51)** de forme conjuguée à la forme locale de la corticale du fémur et une vis **(50)** de mise sous contrainte de traction coopérant, au travers de la cale, avec un taraudage borgne **(43)** présenté par la partie proximale de la tige.

15. Prothèse selon la revendication 14, **caractérisée en ce que** la partie distale de la tige présente une fente **(52)** du type tournevis pour l'adaptation d'un matériel de mise en place/retrait.
